(19) Europäisches Patentamt
European Patent Office
Office européen des brevets

(11) Publication number : **0 581 652 A1**

# (12) EUROPEAN PATENT APPLICATION

(21) Application number : **93401909.2**

(22) Date of filing : **22.07.93**

(51) Int. Cl.$^5$ : **C07H 15/244,** A61K 31/71, C12P 19/56, C12N 1/20, // (C12N1/20, C12R1:04)

(30) Priority : **27.07.92 US 920408**

(43) Date of publication of application : **02.02.94 Bulletin 94/05**

(84) Designated Contracting States : **AT BE CH DE DK ES FR GB GR IE IT LI LU MC NL PT SE**

(71) Applicant : **Bristol-Myers Company**
**345 Park Avenue**
**New York New York 10154 (US)**

(72) Inventor : **Hasegawa, Toshifumi**
**3-6-7, Naka-Machi**
**Houya, Tokyo 202 (JP)**

Inventor : **Yamamoto, Haruaki**
**702-3 Tama**
**Tokyo 206 (JP)**
Inventor : **Kakushima, Masatoshi**
**9-1, 1-204 Ikuta, Tama-ku**
**Isehara, Kanagawa 259-11 (JP)**
Inventor : **Aburaki, Shimpei**
**4-24-1-302 Ikuta, Tama-ku**
**Kawasaki, Kanagawa 214 (JP)**
Inventor : **Furumai, Tamotsu**
**436 Iwai-cho, Hodogaya-ku**
**Yokohama 240 (JP)**

(74) Representative : **Durand, Yves Armand Louis et al**
**CABINET WEINSTEIN 20, Avenue de Friedland**
**F-75008 Paris (FR)**

## (54) Pradimicins T1 and T2 and 11-0-dexylosylpradimicin T1, new members of the pradimicin family of antibiotics.

(57) Novel pradimicin compounds (I) are useful in the treatment of infectious diseases.

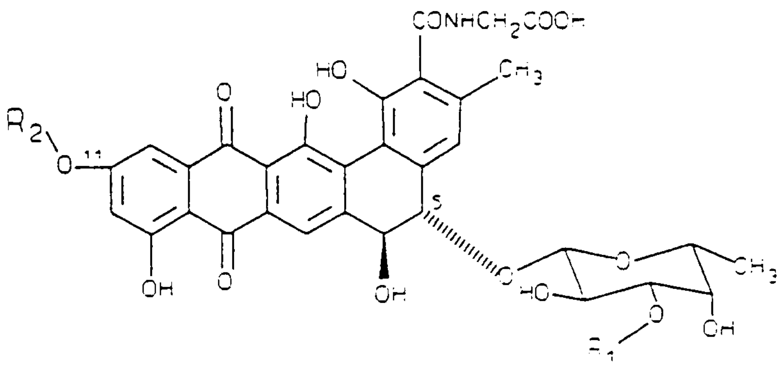

(I)

EP 0 581 652 A1

## Background of the Invention

Various antifungal substances produced by microorganisms have been reported in the literature. However, there are relatively few agents known to be effective against fungi pathogenic to humans, so that a desire still exists for development of antifungal antibiotics which are useful as medicines. Thus, screening programs for microbial products have been operated using devised procedures including unique assay systems and producers.

Pradimicin is a microbial product proved to be effective in protecting mice from lethal systemic infections with Candida albicans, Cryptococcus neoformans and Aspergillus fumigatus, but its poor solubility has necessitated chemical modification of the microbial product.

U.S. patents 5,053,395 and 5,091,418 disclose pradimicin derivatives which are antifungal agents and alpha-glucosidase/inhibitors, respectively.

## Summary of the Invention

The invention is concerned with novel antibiotics which are produced by an Actinomycete strain. The new compounds, which are pradimicins or pradimicin derivatives, are distinct, in both physico-chemical and biological properties from known antibiotics.

## Drawings

Figures 1, 2 and 3 show the UV spectra of Pradimicin T1, Pradimicin T2 and 11-*O*-dexylosylpradimicin T1, respectively.

Figures 4, 5 and 6 depict the IR Spectra of Pradimicin T1, Pradimicin T2 and 11-*O*-dexylosylpradimicin T1, respectively.

Figures 7, 8 and 9 are the $^{1}H$-NMR spectra of pradimicins T1, T2 and 11-*O*-dexylosylpradimicin T1.

Figures 10, 11 and 12 are the $^{13}C$-NMR spectra of pradimicins T1, T2 and 11-*O*-dexylosylpradimin T1.

## Detailed Description of the Invention

Unless otherwise stated, all percentages recited herein are weight percentages, based on total composition weight.

This invention relates to new antibiotic substances, named pradimicins T1 and T2, 11-*O*-dexylosylpradimicin T1, and processes for their preparation by fermentation of Actinomycete strain AA 3798. Also, the use of said compounds in the treatment of infectious diseases caused by microorganisms susceptible to said antibiotics is discussed.

These compounds are water soluble and are new members of the pradimicin family of antibiotics that are different from other known antibiotics in their physico-chemical and biological properties. The following structures were deduced from spectroscopic data and degradation studies.

The features of Structure I are common to all three of the compounds.

Variations of $R_1$ and $R_2$, as indicated below, yield individual structures.

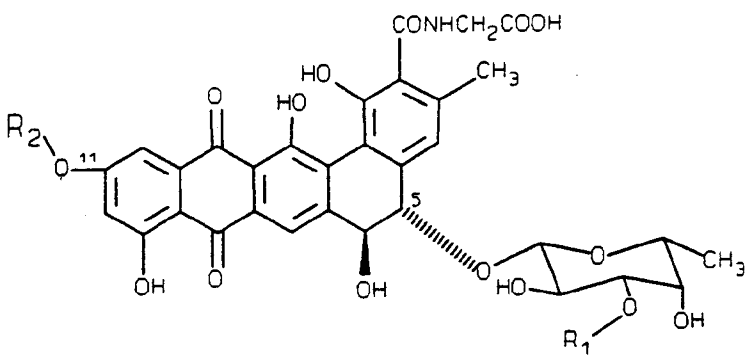

(I)

| | $R_1$ | $R_2$ |
|---|---|---|
| Pradimicin T1 (Ia): | 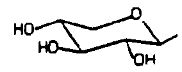 | 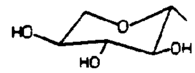 |
| Pradimicin T2 (Ib): | H | 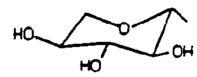 |
| 11-O-Dexylosylpradimicin T1 (Ic): | 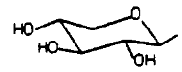 | H |

As part of an effort to screen for water-soluble analogs of pradimicin, applicants discovered that a rare actinomycete numbered AA 3798 produced new members of the pradimicin family of antibiotics. Strain AA 3798 was isolated from a sample of soil picked up at Nerima, Tokyo, Japan on January 29, 1989.

A culture of strain AA 3798 has been deposited with the American Type Culture Collection under the BUDAPEST TREATY ON THE INTERNATIONAL RECOGNITION OF THE DEPOSIT OF MICROORGANISMS FOR THE PURPOSES OF PATENT PROCEDURE, and all restrictions on the availability to the public of deposited microorganism will be irrevocably removed upon the granting of a patent from this application. The deposited culture has been assigned the accession numbered ATCC 55288.

## Taxonomy of Strain AA 3798

The media used in the study on the cultural and physiological characteristics of the strain were prepared according to the recommendation by Shirling and Gottlieb in "Methods for Characterization of *Streptomyces* Species," Intern. J. Syst. Bact., 1966, 16:313-340; by Waksman in The Actinomycetes, Vol. II, "Classification, Identification and Description of Genera and Species", pp. 328-334, published by The Williams and Wilkins Company, Baltimore, 1961; and by Arai in Culture Media for Actinomycetes, published by The Society for Actinomycetes Japan, 1975. Cultures were observed after incubation at 32°C for two to four weeks. Color names and hue numbers (Table 1) are given according to the Manual of Color Names (Japan Color Enterprise Company, Ltd., 1987).

## Morphology

Strain AA 3798 grew well on yeast extract-malt extract agar (ISP-2), inorganic salt-starch agar (ISP-4), yeast starch agar and Bennett's agar at any temperature between 19 and 40°C. Colonies were thickly covered with a powdery to velvety aerial mycelium. The substrate mycelium was well developed and irregularly branched. On some agar media such as water agar, this substrate mycelium had fragmented, irregularly zig-zagged, into short rod elements. The aerial mycelia were abundant and long, moderately branched, straight or irregularly curved. Aerial mycelia were so fine (0.3 μm in diameter) that their sporulation could not be observed under a light microscope, but scanning electron microscopy revealed that they were completely fragmented into spores. The spores were bacillar-shaped and measured 0.3 x 1.0 - 2.0 μm in size, and with a smooth surface. These spores were not motile.

## Cultural characteristics

Strain AA 3798 had white aerial mycelia; upon prolonged incubation, the aerial mycelia sometimes turn pinkish white. The color of vegetative and diffusible pigments on organic media ranged from soft pink to dark red. The color changed from pale reddish yellow to strong yellowish orange by the addition of 0.1N HCl. The cultural characteristics of strain AA 3798 on various media are summarized in Table 1.

| Table 1. Cultural characteristics of strain AA 3798 | | |
|---|---|---|
| Medium | | |
| Sucrose nitrate agar (Waksman med. No. 1) | Growth | Pale reddish yellow (130) |
| | Reverse | Soft yellowish pink (27) |
| | Aerial mycelium | White (388) powdery |
| | Soluble pigment | Soft yellowish pink (28) |
| Glycerol nitrate agar | Growth | Pale reddish yellow (126) |
| | Reverse | Light reddish yellow (131) |
| | Aerial mycelium | White (388) powdery |
| | Soluble pigment | None |
| Glucose asparagine agar (Waksman med. No. 2) | Growth | Light orange (64) |
| | Reverse | Light orange (64) |
| | Aerial mycelium | White (388) powdery |
| | Soluble pigment | None |

| Table 1. (continued) Cultural characteristics of strain AA 3798 | | |
|---|---|---|
| Medium | | |
| Yeast extract-malt extract agar (ISP med. No. 2) | Growth | Dark Red (57) |
| | Reverse | Dark red (57) |
| | Aerial mycelium | Pinkish white (391) powdery, good |
| | Soluble pigment | Dark red (57) |
| Oatmeal agar (ISP med. No. 3) | Growth | Colorless-grayish pink (33) |
| | Reverse | Soft yellowish pink (27) |
| | Aerial mycelium | White (388) powdery |
| | Soluble pigment | Soft pink (25) |
| Inorganic salts-starch agar (ISP med. No. 4) | Growth | Yellowish brown (101) |
| | Reverse | Soft orange (83) |
| | Aerial mycelium | White (388) powdery, good |
| | Soluble pigment | None-beige white (392) |
| Glycerol asparagine agar (ISP med. No. 5) | Growth | Light orange (64) |
| | Reverse | Light orange (65) |
| | Aerial mycelium | White (388) powdery |
| | Soluble pigment | None |
| Tyrosine agar (ISP med. No. 7) | Growth | Light orange (65) |
| | Reverse | Light orange (65) |
| | Aerial mycelium | Beige white (392) powdery |
| | Soluble pigment | None |
| Nutrient agar (Waksman med. No. 14) | Growth | Pale reddish yellow (125) |
| | Reverse | Pale reddish yellow (125) |
| | Aerial mycelium | None |
| | Soluble pigment | None |
| Yeast starch agar | Growth | Dark red (57) |
| | Reverse | Dark red (57) |
| | Aerial mycelium | White (388) powdery, good |
| | Soluble pigment | Strong purplish red (44) |

| Table 1. (continued) Cultural characteristics of strain AA 3798 | | |
|---|---|---|
| Medium | | |
| Bennett's agar (Waksman med. No. 30) | Growth | Dark red (57) |
| | Reverse | Dark red (57) |
| | Aerial mycelium | White (389) powdery, good |
| | Soluble pigment | Strong purplish red (44) |
| Gauze's agar | Growth | Pale reddish yellow (125) |
| | Reverse | Light orange (64) |
| | Aerial mycelium | White (388) powdery, good |
| | Soluble pigment | Pinkish white (391) |
| Peptone corn agar | Growth | Grayish pink (32) |
| | Reverse | Grayish pink (32) |
| | Aerial mycelium | White (389) powdery, scant |
| | Soluble pigment | Soft pink (25) |

Physiological characteristics

The physiological characteristics and utilization of carbon sources of strain AA 3798 are shown in Tables 2 and 3, respectively. No vitamins were essential for growth.

| Table 2. Physiological characteristics of strain AA 3798 | |
|---|---|
| Test | Results |
| Starch hydrolysis (on ISP med. No. 4) | Positive |
| Nitrate reduction (Difco, nitrate broth) | Negative |
| Milk (Difco, 10% skimmed milk)<br>Coagulation<br>Peptonization | <br>Positive<br>Positive |
| Cellulose decomposition (sucrose nitrate solution with a paper strip as the sole carbon source) | Negative |
| Gelatin liquefaction<br>On plain gelatin<br>On glucose peptone gelatin | <br>No growth<br>No growth |
| Melanin formation (On ISP med. No. 7) | Negative |

| Table 2. (cont'd) Physiological characteristics of strain AA 3798 | |
|---|---|
| Test | Results |
| Temperature range for growth (°C)<br>Optimum temperature (°C)<br>(On Yeast starch agar) | 19 - 40<br>29 - 34 |
| pH range for growth<br>Optimum pH<br>(On Trypticase soy broth, BBL) | 6 - 8<br>7 - 8 |
| Growth in/at<br>Lysozyme 0.01%<br>0.1%<br>NaCl 2%<br>8% | <br>Positive<br>Negative<br>Positive<br>Negative |

| Table 3. Utilization of carbon sources by strain AA 3798 | |
|---|---|
| Carbon Source | Growth |
| Arabinose | ++ |
| Inositol | ++ |
| D-Mannitol | ++ |
| Sucrose | ++ |
| L-Rhamnose | ++ |
| Raffinose | ++ |
| D-Xylose | ++ |
| D-Glucose | ++ |
| Fructose | + |

+: moderate growth, ++: abundant growth

Antibiotic susceptibility of strain AA 3798 was examined using antibiotic disks (Tridisk, Eiken Chemical Co., Ltd). The disks were placed onto the surface of yeast-glucose-malt agar (yeast extract 0.1%, glucose 1%, malt extract 0.1%, $CaCl_2 \cdot 2H_2O$ 0.05%, and agar 1.5%) which had been seeded by strain AA 3798 (4% inoculum), and the plates were then incubated at 37°C for 4 days.

Strain AA 3798 was resistance to 20 μg of ampicillin, 10 μg of cephalexin, 50 μg of fosfomycin, 15 μg of lincomycin, 5 μg of gentamicin, 10 μg of tobramycin, 15 μg of nalidixic acid, 300 U of polymyxin B, 10 μg of erythromycin and 15 μg of josamycin. It was susceptible to 1 μg of clavulanic acid, 2 μg of ticarcillin, 30 μg of tetracycline, 10 μg of chloramphenicol, 30 μg of kanamycin, 2 μg of norfloxacin and 50 U of colistin.

## Cell wall chemotype

Whole-cell compositions were analyzed by the procedures described by Becker and Lechevalier in "Rapid Differentiation between *Nocardia* and *Streptomyces* by Paper Chromatography of Whole Cell Hydrolysate," Appl. Microbiol., 1964, 12:421-423, and in "Chemical Compositions of Cell-Wall Preparations from Strains of Various Form-Genera of Aerobic Actinomycetes," Appl. Microbiol., 1965, 13:236-243. Strain AA 3798 contained meso-diaminopimeric acid, glucose, glucosamine, galactose and small amounts of ribose, mannose and xylose, but madurose and arabinose were not detected, indicating that the cell wall of this strain is type IIIC. Mycolic acids were not detected by the method of Minnikin et al in "Differentiation of *Mycobacterium, Nocardia,* and Related Taxa by Thin-Layer Chromatographic Analysis of Whole-Organism Methanolysates," J. Gen. Microbiol., 1975, 88:200-204. Analysis of the menaquinone composition using the procedure of Collins *et al* in "A Note on the Separation of Natural Mixtures of Bacterial Menaquinones Using Reverse-Phase Thin-Layer Chromatography," J. Appl. Bacteriol., 1980, 48:277-282, revealed 69% MK-9($H_8$), 18% MK-9($H_6$), 6% MK-9($H_4$) and 6% MK-9($H_{10}$). The whole cell fatty acids determined by the method of Suzuki *et al* in "Taxonomic Significance of Cellular Fatty Acid Composition in Some Coryneform Bacteria," Int. J. Syst. Bacteriol., 1983, 33:188-200, consisted of 34% 14-methylpentadecanoic acid (*iso*16:0), 21% 14-methylhexadecanoic acid (*anteiso*-17:0), 14% 15-methylhexadecanoic acid (*iso*17:0) and 10% 10-methyloctadecanoic acid (10Me18:0).

According to the guide to generic identification of Actinomycetes described by H.A. Lechevalier (in Bergey's Manual of Systematic Bacteriology, Vol. 4., Eds. S.T. Williams et al, pp. 2344-2347, 1989, Williams & Wilkins),

the presence of meso-diaminopimelic acids together with no diagnostic sugar and long chains of spores formed on the aerial hyphae indicate that strain AA 3798 belongs to the genus *Nocardiopsis* as only one generic possibility. However, on the basis of the definition described by Grund and Kroppenstedt ("Fermentation, Chemotaxonomy and Numerical Taxonomy of the Genus *Nocardiopsis* Meyer", 1976, Int. J. Syst. Bacteriol. 40:5-11, 1990), the major components of both menaquinones and fatty acids from strain AA 3798 are quite different from those of genus *Nocardiopsis.* Since no actinomycetous organisms with the cell wall chemotype as described above have been reported previously, it is impossible to assign the genus of strain AA 3798 at present, although the proposal of a new genus might be justified. Thus, strain AA 3798 was assigned as an unidentified actinomycete.

The novel antibiotics of this invention are produced during the aerobic fermentation of suitable liquid nutrient media under controlled conditions via inoculation with the above strain or its variants or mutants. Liquid nutrient media such as those employed for the production of common antibiotics are suitable for producing pradimicins T1 and T2. Desirable carbon sources are glucose, glycerol, fructose, sucrose, xylose, starch and other carbohydrates and preferable nitrogen sources are soybean meal, cotton seed meal, peptone, meat extract, fish meals, corn steep liquor and the like. Desirable inorganic salts are cobalt chloride, calcium carbonate and potassium phosphate, which are added if necessary. A preferable liquid medium is the one described in Example 2. Another more convenient medium is FR-22, which is composed of soybean meal (1-3%), glucose (1-3%), D-aspartic acid (0.1-0.2%) and $CaCO_3$(0.05-0.3%). Adekanol, silicone and the like are used as the antiforming agents. As every microbiologist who is experienced in fermentation will appreciate, the production and ratio of pradimicins T1 and T2 vary according to the fermentation conditions and the strains and/or media used. Preferred fermentation conditions are aerobic cultivations at pH 5-8 at 20-37°C for 5-14 days, preferably at pH 6-7 at 28-34°C for 6-12 days. Production of pradimicins T1 and T2 can be readily monitored during the course of the fermentation by conventional techniques such as thin layer chromatography, HPLC, visible absorption and anti-*Candida* activity.

Isolation and purification

For the isolation of pradimicins T1 and T2 from the fermentation broth, separation methods which are usually used to isolate metabolites produced by microorganisms from their cultured broth can properly be used. As pradimicins T1 and T2 are water-soluble acidic substances and predominantly produced in the fermentation broth, they are recovered advantageously by usual procedures for hydrophilic acidic substances such as organic solvent extraction, ion exchange resin, acidic precipitation, partition chromatography and the like; either alone or in combination. For example, after completion of the fermentation, the whole fermentation broth is centrifuged or filtered. The resulting supernatant or filtrate is adsorbed on high porous polymer resin such as Diaion HP-20 (Mitsubishi Kasei Co.) and eluted with water miscible organic solvent such as methanol acetonitrile or acetone. The eluate is concentrated *in vacuo.* Precipitation in acetone or lyophilization gave crude pradimicins T1 and T2. For purification, the crude material is applied onto a reverse phase silica gel column such as YMC-ODS A60 (Yamamura Chemical Lab.) and eluted with acetonitrile:0.15% phosphate buffer, pH 3.5, (14.5:85.5, V/V), yielding pure pradimicins T1 and T2.

Pradimicin T1 may be converted to its 11-*0*-dexylosyl derivative. Heating pradimicin T1 in an alkaline medium for a period sufficient to cleave the xylosyl group at the 11-*0*-position provides the corresponding 11-*0*-dexylosyl derivative. The solvent used may be for example dioxane, tetrahydrofuran, water, lower alkanol, or mixtures thereof; alkaline hydroxide may be for example sodium hydroxide, potassium carbonate, lithium hydroxide. The temperature may be from about 60°C to about 100°C, or the reflux temperature of the solvent. Reaction time may be from about 1 to 10 hrs and will depend on the reaction conditions employed.

Physico-chemical properties

Physico-chemical properties of pradimicins T1 and T2, and 11-*0*-dexylosylpradimicin T1 are summarized in Table 4.

| Table 4. Physico-chemical properties of pradimicins T1 and T2, 11-O-dexylosylpradimicin T1 | | | |
|---|---|---|---|
| | Pradimicin T1 | Pradimicin T2 | 11-O-dexylosyl-Pradimicin T1 |
| Appearance: | Dark red amorphous | Dark red amorphous | Dark red amorphous |
| M.P. (dec.): | 190-195°C | 185-190°C | 200-210°C |
| $[\alpha]_D^{30}$ (c 0.05, 0.1N HCl): | Unable to determine | Unable to determine | Unable to determine |
| FAB-MS (m/z): | $932(M + H)^+$ | $800(M + H)^+$ | $800(M + H)^+$ |
| Molecular formula: | $C_{42}H_{45}NO_{23}$ | $C_{37}H_{37}NO_{19}$ | $C_{37}H_{37}NO_{19}$ |
| UV $\lambda_{max}$ nm (ε) | | | |
| in 0.02N NaOH-MeOH(1:1): | 243(33,600)<br>316(14,000)<br>500(14,200) | 242(29,500)<br>316(12,200)<br>499(12,200) | 246(32,500)<br>308(21,300)<br>505(15,300) |
| in 0.02N HCl-MeOH(1:1): | 232(33,100)<br>290(25,300)<br>457(11,000) | 233(29,600)<br>290(22,600)<br>456(9,800) | 232(28,500)<br>299(23,400)<br>459(8,800) |
| in MeOH-$H_2O$(1:1): | 224(30,400)<br>274(25,100)<br>493(10,100) | 220(27,800)<br>274(23,500)<br>501(9,800) | 230(31,800)<br>288(23,800)<br>477(9,500) |
| Solubility in solvent: | | | |
| Soluble: | Dimethyl sulfoxide, N,N-dimethylformamide and alkaline water, etc. | | |
| Slightly soluble: | Ethanol, methanol and acetone, etc. | | |
| Insoluble: | Benzene, chloroform, etc. | | |
| TLC ($SiO_2$), Rf: | | | |
| n-BuOH-AcOH-$H_2O$=2:1:1 | 0.48 | 0.50 | 0.70 |
| n-BuOH-AcOH-Pyridine-$H_2O$=6:1:4:3 | 0.28 | 0.38 | 0.57 |
| HPLC Rt (min) (ODS, $CH_3CN$-0.15% $KH_2PO_4$, pH 7.0 = 12:88 v/v) | 7.5 | 4.1 | 11.0 |

UV, IR, $^1H$ and $^{13}C$ NMR spectra of pradimicins T1 and T2 and 11-*O*-dexylosylpradimicin T1 are shown in Figures 1 through 12, respectively.

Biological properties

The *in vitro* antifungal activities of pradimicins T1 and T2 and 11-*O*-dexylosylpradimicin T1 were determined by the agar dilution method on yeast morphology agar containing 1/15M phosphate buffer (pH 7.0). The MICs for individual isolates are shown in Table 5.

| Table 5. In vitro antifungal activity | | |
|---|---|---|
| Test organism | MIC (μg/ml) | |
| Saccharomyces cerevisiae ATCC 9763 | Pradimicin T1<br>Pradimicin T2 | 3.1<br>0.8 |
| | 11-O-Dexylosyl-pradimicin T1 | 3.1 |
| | Amphotericin B | 0.4 |
| | Ketoconazole | 50 |
| Candida albicans A9540 | Pradimicin T1<br>Pradimicin T2 | 6.3<br>3.1 |
| | 11-O-Dexylosyl-pradimicin T1 | 6.3 |
| | Amphotericin B | 0.4 |
| | Ketoconazole | 50 |
| Candida albicans ATCC 38247 | Pradimicin T1<br>Pradimicin T2 | 1.6<br>1.6 |
| | 11-O-Dexylosyl-pradimicin T1 | 3.1 |
| | Amphotericin B | 3.1 |
| | Ketoconazole | 25 |
| Candida albicans ATCC 32354 (B311) | Pradimicin T1<br>Pradimicin T2 | 6.3<br>3.1 |
| | 11-O-Dexylosyl-pradimicin T1 | 3.1 |
| | Amphotericin B | 0.4 |
| | Ketoconazole | 50 |

| Table 5. (continued) in vitro antifungal activity | | |
|---|---|---|
| Test organism | MIC (μg/ml) | |
| *Candida albicans* 83-2-14 (Juntendo) | Pradimicin T1<br>Pradimicin T2 | 6.3<br>3.1 |
| | 11-O-Dexylosyl-pradimicin T1 | 3.1 |
| | Amphotericin B | 0.4 |
| | Ketoconazole | 12.5 |
| *Candida tropicalis* 85-8 (Kitasato) | Pradimicin T1<br>Pradimicin T2 | 25<br>6.3 |
| | 11-O-Dexylosyl-pradimicin T1 | 12.5 |
| | Amphotericin B | 0.8 |
| | Ketoconazole | 100 |
| *Candida tropicalis* IFO 10241 | Pradimicin T1<br>Pradimicin T2 | 12.5<br>6.3 |
| | 11-O-Dexylosyl-pradimicin T1 | 12.5 |
| | Amphotericin B | 0.8 |
| | Ketoconazole | 50 |
| *Cryptococcus neoformans* D49 | Pradimicin T1<br>Pradimicin T2 | 3.1<br>6.3 |
| | 11-O-Dexylosyl-pradimicin T1 | 6.3 |
| | Amphotericin B | 0.4 |
| | Ketoconazole | 0.8 |
| *Cryptococcus neoformans* IAM 4514 | Pradimicin T1<br>Pradimicin T2 | 3.1<br>6.3 |
| | 11-O-Dexylosyl-pradimicin T1 | 6.3 |
| | Amphotericin B | 0.4 |
| | Ketoconazole | 0.4 |

| Table 5. In vitro antifungal activity | | |
|---|---|---|
| Test organism | MIC (μ g/ml) | |
| Aspergillus fumigatus IAM 2034 | Pradimicin T1<br>Pradimicin T2 | 6.3<br>>100 |
| | 11-O-Dexylosyl-pradimicin T1 | 25 |
| | Amphotericin B | 0.8 |
| | Ketoconazole | 3.1 |
| Trichophyton mentagrophytes 4329 | Pradimicin T1<br>Pradimicin T2 | 6.3<br>50 |
| | 11-O-Dexylosyl-pradimicin T1 | 12.5 |
| | Amphotericin B | 0.8 |
| | Ketoconazole | 0.8 |

Medium: Yeast morphology agar + 1/15M phosphate buffer (pH 7.0)
Inoculum: $10^6$ cells/ml (except *T. mentagrophytes*: $10^7$ cells/ml)
Incubation conditions: 28°C, 40 hrs. (60 hrs. *T. mentagrophytes*)

Pradimicins T1 and T2 were evaluated for *in vivo* efficacy against a *Candida albicans* A9540 systemic infection in male ICR mice (20-24 g body weight) as described by Oki *et al.* in water-soluble pradimicin derivatives, synthesis and antifungal evaluation of N,N,-dimethyl pradimicins, J. Antibiotics 43:1230-1235, 1990. Five mice at each dose level were challenged intravenously with ten times the 50% lethal dose of the pathogen suspended in saline. Antibiotics were intravenously administered once immediately after the fungal challenge. Infection control animals (n=10) died from 7 to 12 days. The 50% protective dose ($PD_{50}$) was calculated from the survival rate 20 days after the fungal infection. The acute toxicity of pradimicins T1 and T2 was determined in mice after a single intravenous dose. The $PD_{50}$ and $LD_{50}$ of pradimicins T1 and T2 are presented in Table 6.

| Table 6. In vivo activity against C. albicans A9540 systemic infection in mice | | |
|---|---|---|
| Compound | $PD_{50}$ mg/kg | $LD_{50}$ mg/kg |
| Pradimicin T1<br>Pradimicin T2 | 27<br>>50 | 300<br>>600 |
| Amphotericin B | 0.2 | 4.5 |
| Ketoconazole | >50 | NT |

NT: Not tested

## Examples

### Compositions

Compositions employing one or more of the compounds of the invention and/or their acid or base addition salts may contain suitable amounts of pharmaceutically acceptable excipients.

By "acid or base addition salts", applicants mean compounds or complexes formed when the subject compounds are contacted with acidic or basic reagents conventionally employed to yield bioavailable forms of therapeutic agents. Suitable reagents include hydrochloric and, oxalic acid, sodium hydroxide, potassium hydroxide, lithium hydroxide, sodium carbonate and the like.

The compounds of the invention will generally be present in formulations with about 0.001 to about 99.99% of one or more excipients in formulations to be administered via oral, buccal, nasal, topical or parenteral dosage forms. Parenteral dosage forms are preferred. Useful excipients include carriers, colorants, perfumes, flow control agents, stabilizers and the like. The content of drug in formulations usually 50 to about 90 wt %. Useful

amounts of the therapeutic agents range from about 5 mg/kg to about 100 mg/kg of patient body weight.

Suitable "patients" include mammals, preferably humans, and a variety of other organisms, such as Canitadida species, Cryptoitacoccus neofoitarmans and Aspeitargillus famiitagatus.

Example 1. Seed culture

An actinomycete strain AA 3798 was propagated on YSM agar slant composed of soluble starch 1%, yeast extract (Difco laboratories) 0.1%, soytone (Difco laboratories) 0.1%, $CaCl_2 \cdot 2H_2O$ 0.05% and agar 1.6%, at 32°C for 20 days. A portion of the mature agar slant was inoculated into a 500-ml Erlenmeyer flask containing 100 ml of the medium V15-1 composed of glucose 1.5%, peptone (Daigo Eiyo Co.) 0.2%, NZ-case (Scheffield Products) 0.2%, meat extract (Mikuni Kogyo Co.) 1.0%, $NaNO_3$ 0.2%, $K_2HPO_4$ 0.1%, $MgSO_4 \cdot 7H_2O$ 0.05%, and $CaCl_2 \cdot 2H_2O$ 0.05% and then incubated at 32°C for five days on a rotary shaker with orbiting at 200 rpm. The resulting vegatative mycelia were washed and resuspended with a half volume of 20% aq. glycerol and then stored at -80°C. The seed culture for flask fermentation was prepared by inoculating 1 ml of frozen vegetative mycelia in 100 ml of the medium V15-1 in a 500-ml Erlenmeyer flask and shaking for four days at 32°C.

Example 2. Flask fermentation

Each 5-ml portion of the seed culture as set forth in Example 1 was transferred into 500-ml Erlenmeyer flasks containing 100 ml of production medium 84B composed of soybean meal (Nikko Seiyu Co.) 3%, Pharmamedia (Trader's Protein) 0.5%, glucose 3%, yeast extract (Oriental Yeast Co.) 0.1% and $CaCO_3$ 0.3%. The pH was adjusted to 7.0 before autoclaving. The fermentation was conducted on a rotary shaker operating at 200 rpm for 12 days at 28°C. The production of the antibiotics was monitored by measuring the visible absorption at 500 nm in 0.02N NaOH:MeOH (1:1) solution. For quantitative analysis of pradimicins T1 and T2, the supernatant was diluted ten fold with dimethylsulfoxide, and filtered (Ekicrodisc 13CR, Pore size: 0.45 μm, Gelman Science Japan, Ltd.), and the filtrate (10 μl) was analyzed by HPLC on Excel pak SIL-C185R (Yokogawa Electronic Co., Ltd.) using acetonitrile: 0.15% phosphate buffer, pH 3.5 (19:81) at a flow rate of 1 ml/min with 460 nm detection. The total titer of pradimicins T1 and T2 reached about 900 μg/ml after 12 days of fermentation (pradimicin T1: 680 μg/ml, pradimicin T2: 230 μg/ml).

Example 3. Alternative flask fermentation

Each 5-ml portion of the seed culture as set forth in Example 1 was transferred into 500-ml Erlenmeyer flasks containing 100 ml of the production medium, named FR-22, which was composed of defatted soybean meal 3%, glucose 3%, D-aspartic acid 0.2% and $CaCO_3$ 0.3% (pH was adjusted to 7.0 before autoclaving). The fermentation was carried out on a rotary shaker at 28°C for eight days, during which the production of the antibiotic reached about 1,400 μg/ml (pradimicin T1: 820 μg/ml, pradimicin T2: 520 μg/ml).

Example 4. Isolation of pradimicins T1 and T2

The fermentation broth as set forth in Example 2 was centrifuged at 3,000 rpm for 20 min. The supernatant (3.5 L) was adsorbed on a column (2.2 L) of Diaion HP-20. The resin was eluted with 40% aq. methanol (5.3 L) after washing with water (7 L) and 20% aq. methanol (5 L). The eluate was concentrated *in vacuo* and lyophilized to yield a crude solid (5.9 g). This solid (4.9 g) was dissolved in water (2 L), the pH was adjusted to 2.0 with 1N HCl, and the product was extracted with n-butanol (2 L). The organic layer was back-extracted with alkaline water (pH 8.5, 0.6 L) and the aqueous layer was concentrated after adjusting to pH 7.0. The aqueous residue (0.2 L) was dropwise added to acetone (1.8 L) and the resulting dark red powder (2.5 g) was collected by filtration. This powder (2.4 g) was dissolved in a mixture of acetonitrile: 0.15% phosphate buffer, pH 3.5, (14.5:85.5, 240 ml) and then subjected to chromatography on a column of YMC-gel, ODS-A60 (10 L) which had been equilibrated with the same solvent. The eluates with the same solvent were monitored by HPLC (column: YMC A30, 4.5 mm ID x 100 mm, 3 μm, Yamamura Chemical Lab., mobile phase: acetonitrile: 0.15% phosphate buffer, pH 3.5, (19:81)). The fractions either containing pradimicin T1 or pradimicin T2 were pooled, concentrated and then desalted to afford pure pradimicin T1 (293 mg) and pradimicin T2 (79 mg).

Example 5. Preparation of 11-0-Dexylosylpradimicin T1

A solution of pradimicin T1 (500 mg) in 80 ml of 1N sodium hydroxide was heated at 65°C for seven hours. The reaction mixture was cooled, adjusted to pH 3.0 with 6N HCl, and the solid formed was collected by centrifugation (3,000 rpm). The solid was charged on a column of YMC gel ODS-A60 (1,350 ml) and eluted with $CH_3CN$: 0.15% phosphate buffer, pH 7.0 (7:93, 15L). The fractions containing 11-*0*-dexylosylpradimicin T1 were pooled, concentrated *in vacuo* and passed through a short column of Diaion HP-20. The column was washed with water and eluted with 60% aqueous acetone (500 ml). Concentration of the eluate *in vacuo* and lyophilization from water gave a dark red powder (294 mg, 68%).

## Claims

1. A compound of structure I:

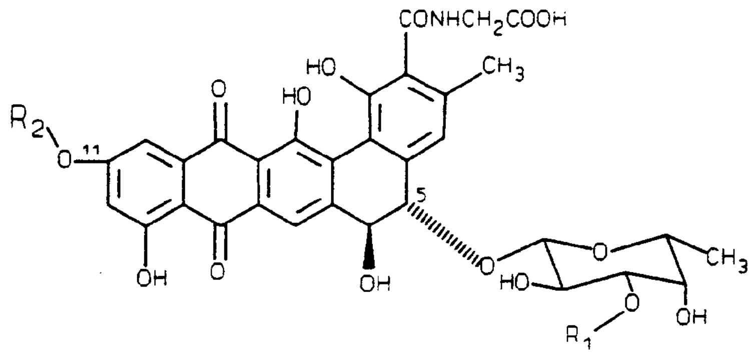

(I)

wherein $R_1$ is each H or

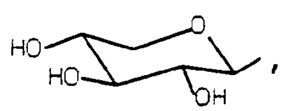

and
$R_2$ is

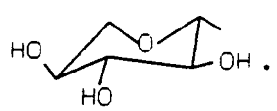

2. The acid or base addition products of the compound of claim 1.

3. The compound of claim 1 wherein $R_1$ is

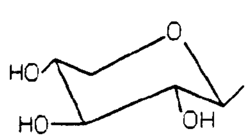

and $R_2$ is

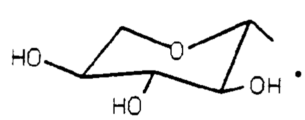

4. The compound of claim 1 wherein $R_1$ is H and $R_2$ is

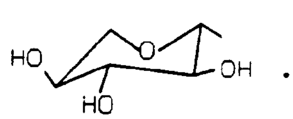

5. The compound of claim 1 wherein $R_1$ is

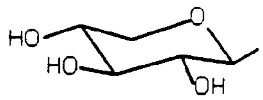

and $R_2$ is H.

6. A pharmaceutical composition comprising an effective antibiotic amount of at least one compound of claim 1 and one or more pharmaceutically acceptable excipients.

7. A biologically pure culture of the microorganism Actinomycete strain AA 3798 (ATCC 55288), said culture being capable of producing at least one compound of claim 1.

8. A biologically pure concentrate containing a carrier and the compound of claim 1.

9. Use of an antibiotic effective amount of at least one compound of claim 1 in the manufacture of a medicament for treating infectious diseases in a patient afflicted with such diseases.

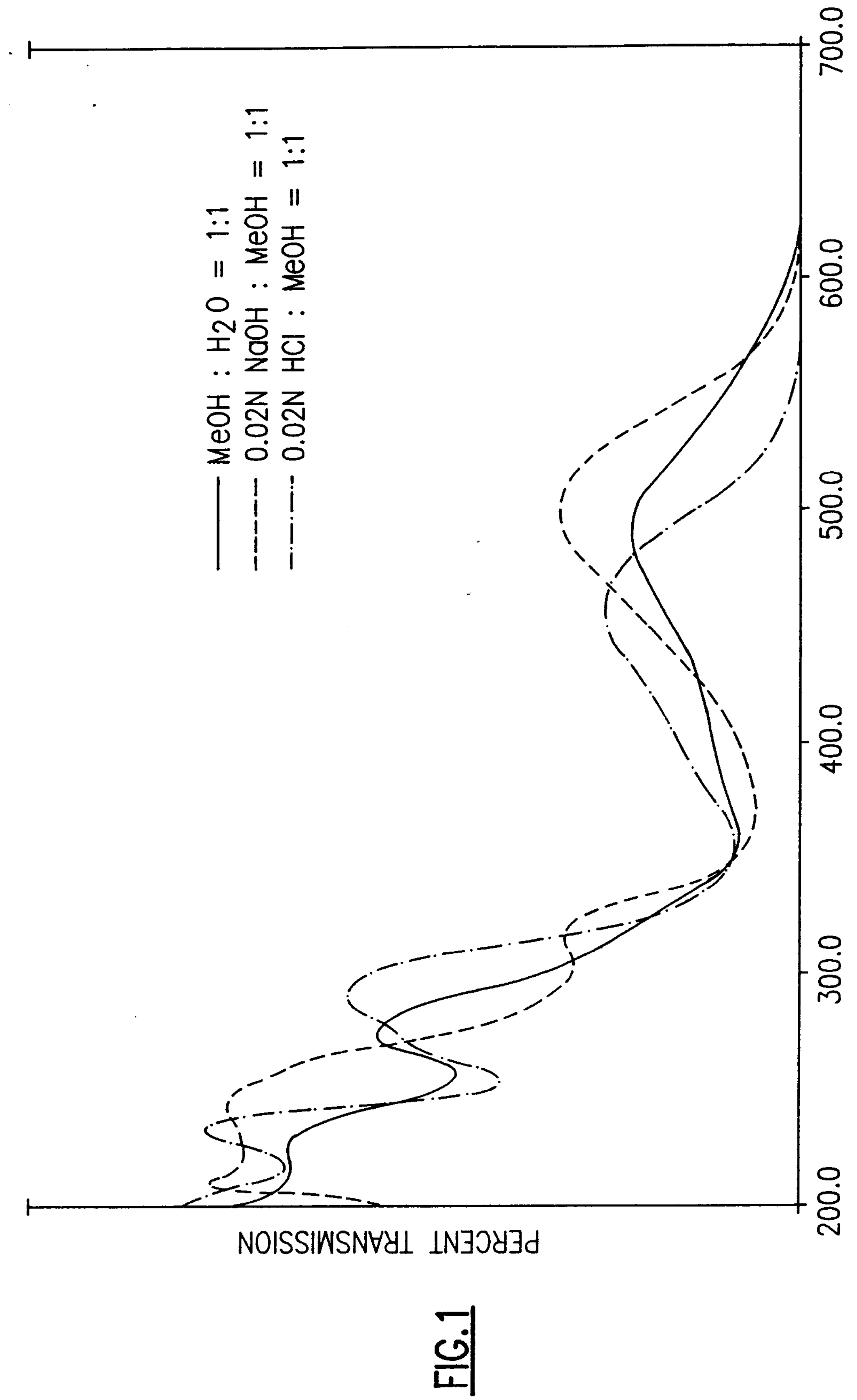
MeOH : $H_2O$ = 1:1
0.02N NaOH : MeOH = 1:1
0.02N HCl : MeOH = 1:1
PERCENT TRANSMISSION
200.0
300.0
400.0
500.0
600.0
700.0
WAVELENGTH IN MILLIMICRONS

FIG.1

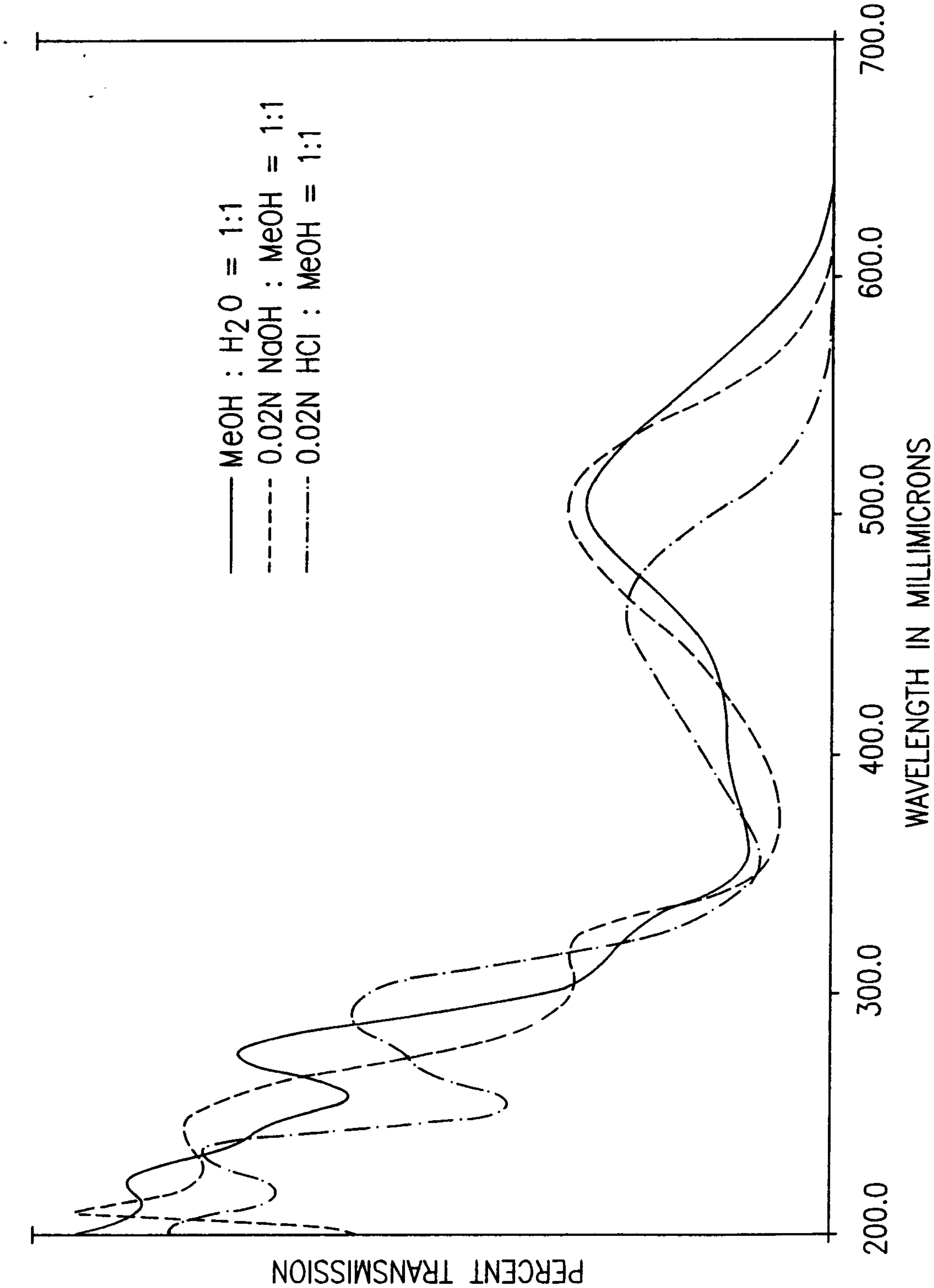
MeOH : $H_2O$ = 1:1
0.02N NaOH : MeOH = 1:1
0.02N HCl : MeOH = 1:1
PERCENT TRANSMISSION
WAVELENGTH IN MILLIMICRONS
200.0
300.0
400.0
500.0
600.0
700.0

FIG.2

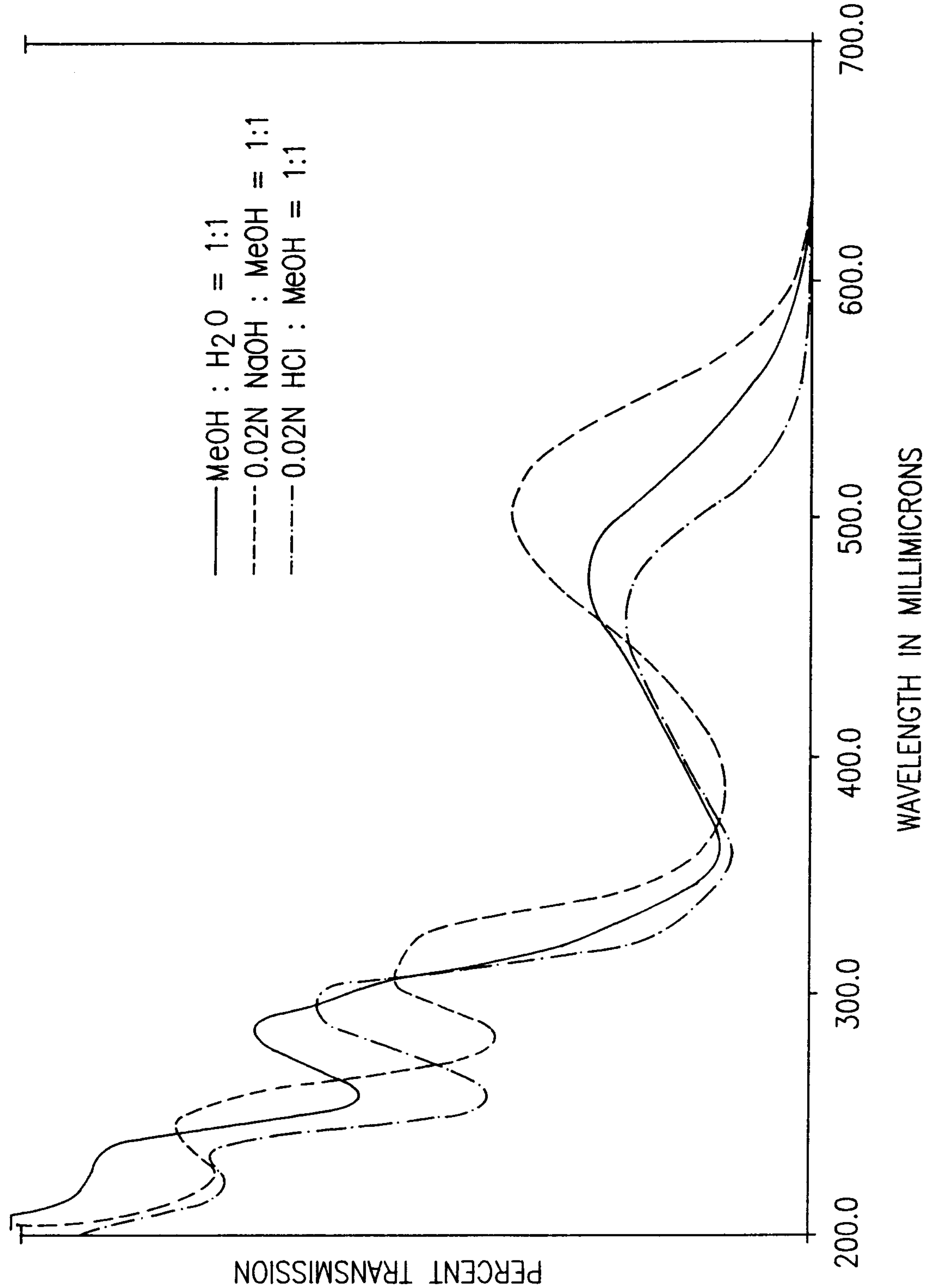
MeOH : $H_2O$ = 1:1
0.02N NaOH : MeOH = 1:1
0.02N HCl : MeOH = 1:1
PERCENT TRANSMISSION
200.0
300.0
400.0
500.0
600.0
700.0
WAVELENGTH IN MILLIMICRONS

FIG.3

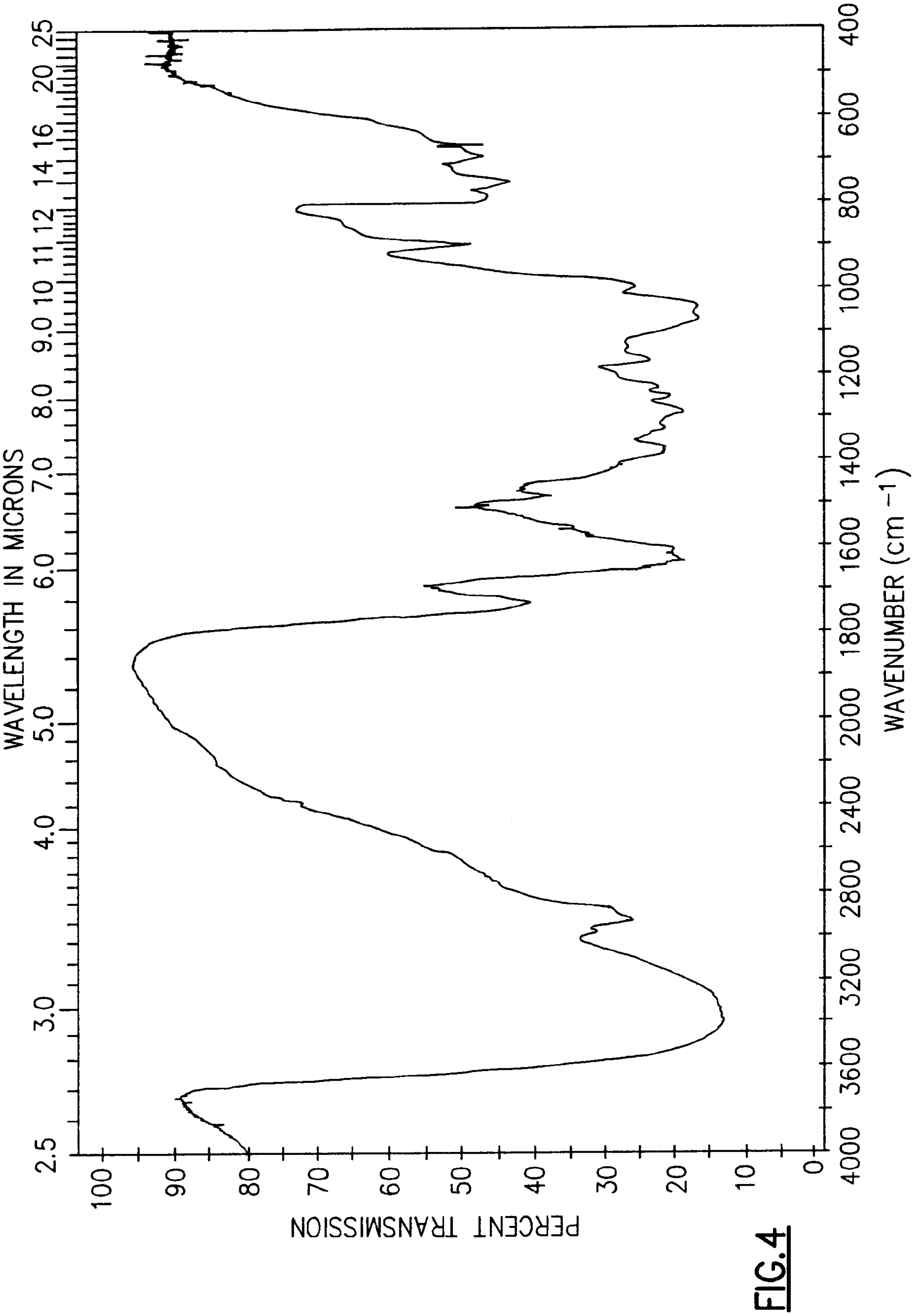
WAVELENGTH IN MICRONS
2.5
3.0
4.0
5.0
6.0
7.0
8.0
9.0
10
11
12
14
16
20
25
PERCENT TRANSMISSION
0
10
20
30
40
50
60
70
80
90
100
WAVENUMBER (cm −1)
4000
3600
3200
2800
2400
2000
1800
1600
1400
1200
1000
800
600
400

FIG.4

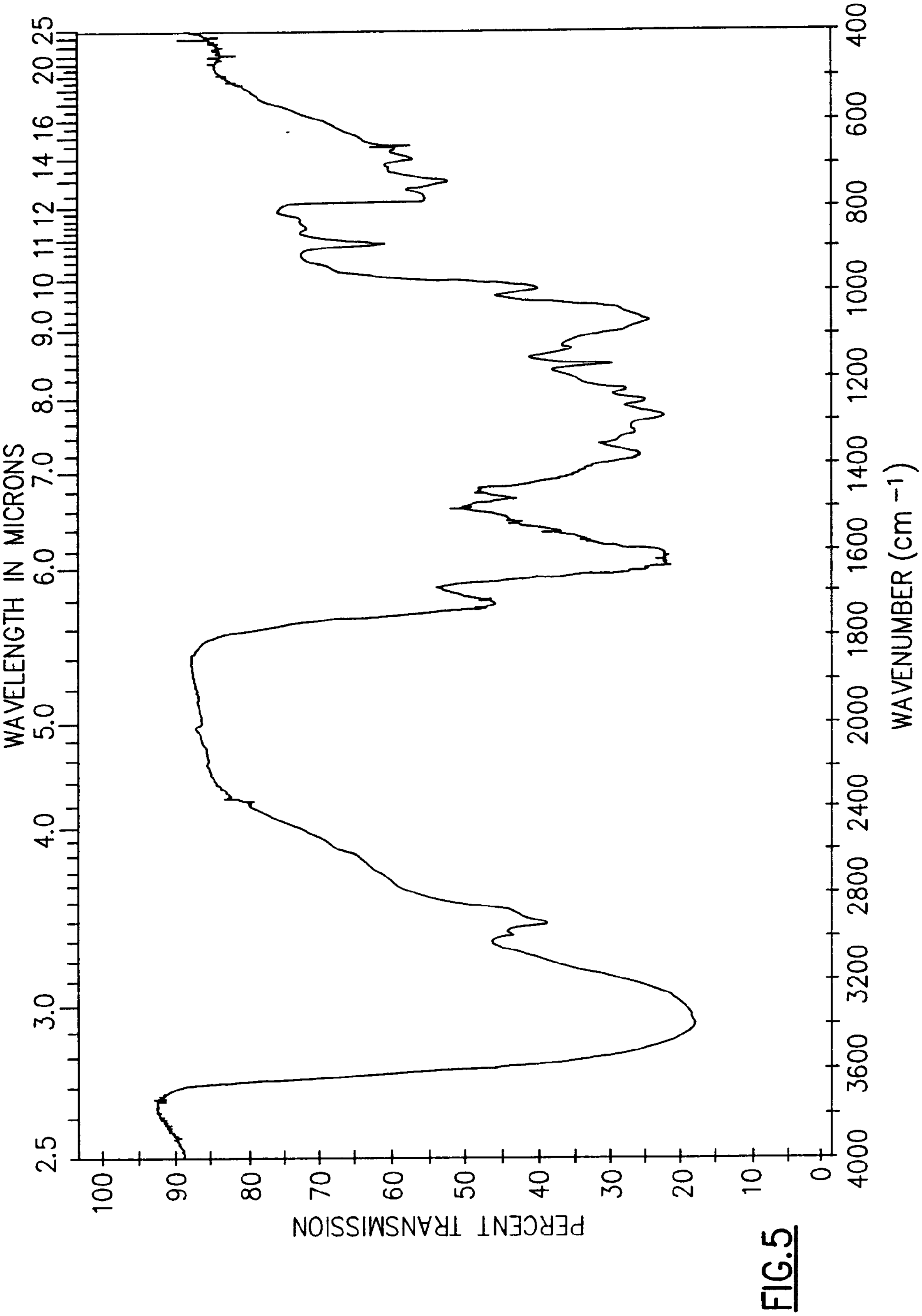
WAVELENGTH IN MICRONS
2.5
3.0
4.0
5.0
6.0
7.0
8.0
9.0 10 11 12 14 16 20 25
PERCENT TRANSMISSION
100
90
80
70
60
50
40
30
20
10
0
4000
3600
3200
2800
2400
2000
1800
1600
1400
1200
1000
800
600
400
WAVENUMBER (cm −1)

FIG.5

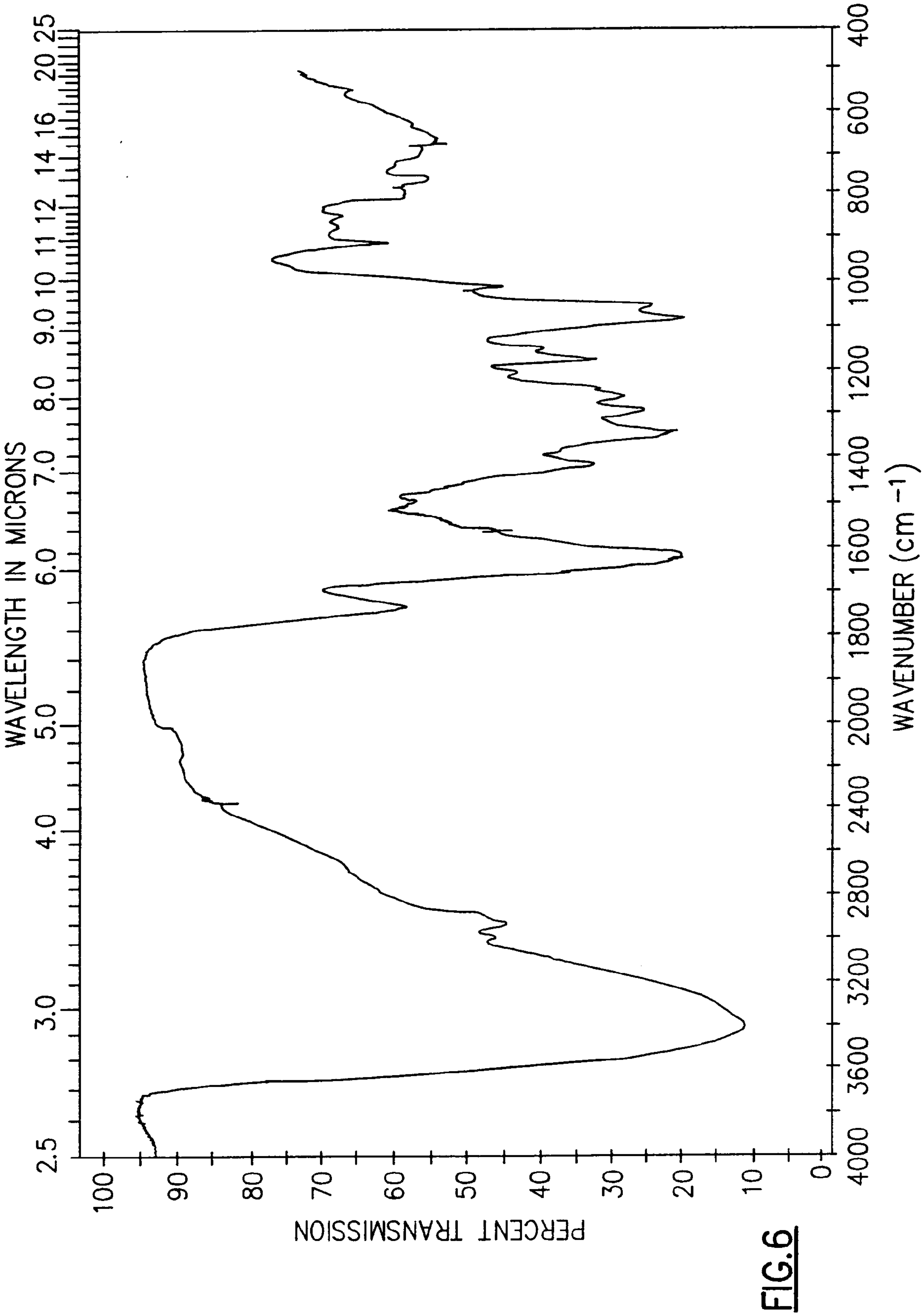

WAVELENGTH IN MICRONS
2.5
3.0
4.0
5.0
6.0
7.0
8.0
9.0
10
11
12
14
16
20
25
PERCENT TRANSMISSION
100
90
80
70
60
50
40
30
20
10
0
WAVENUMBER (cm $^{-1}$)
4000
3600
3200
2800
2400
2000
1800
1600
1400
1200
1000
800
600
400


FIG.6

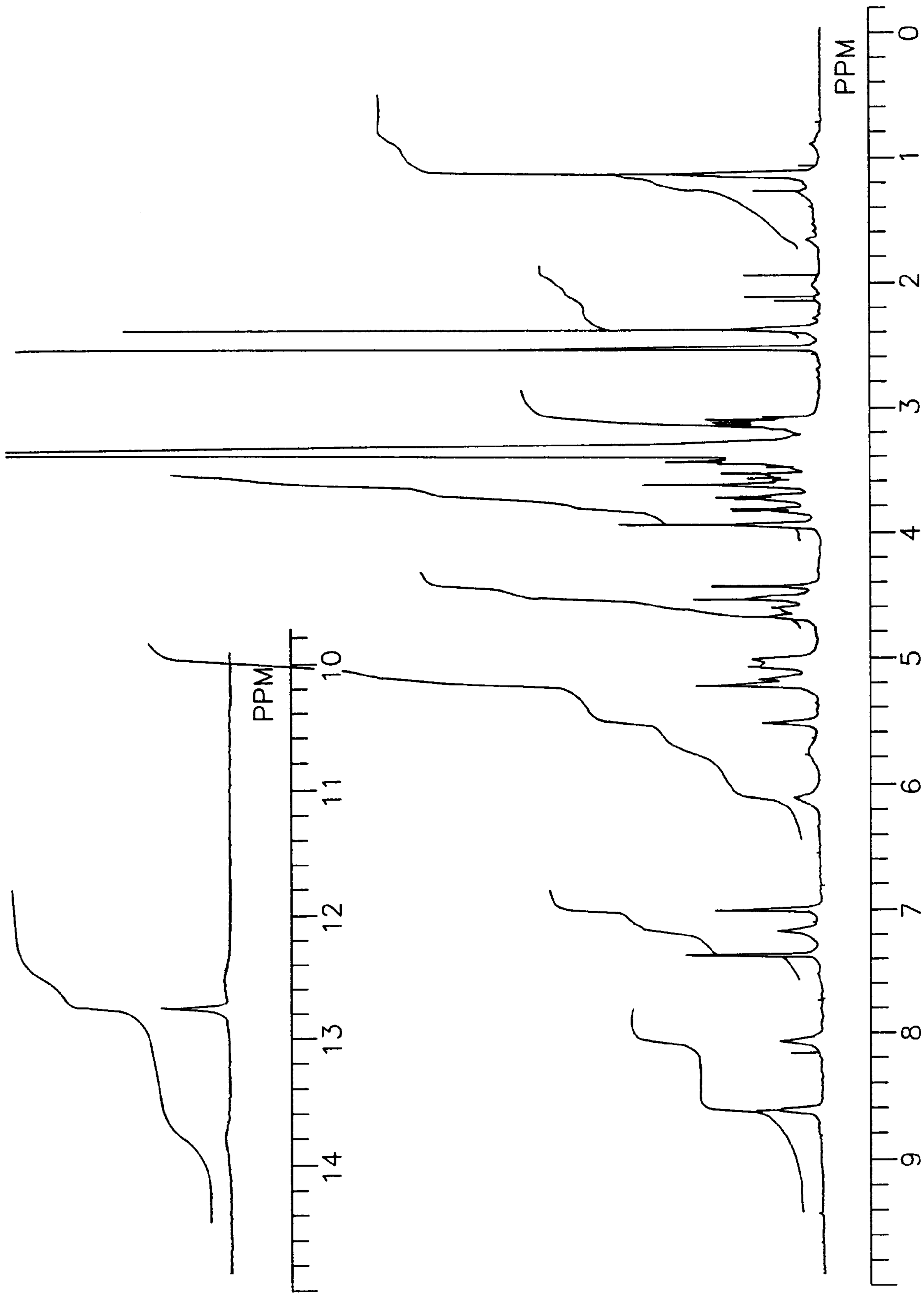


FIG.7

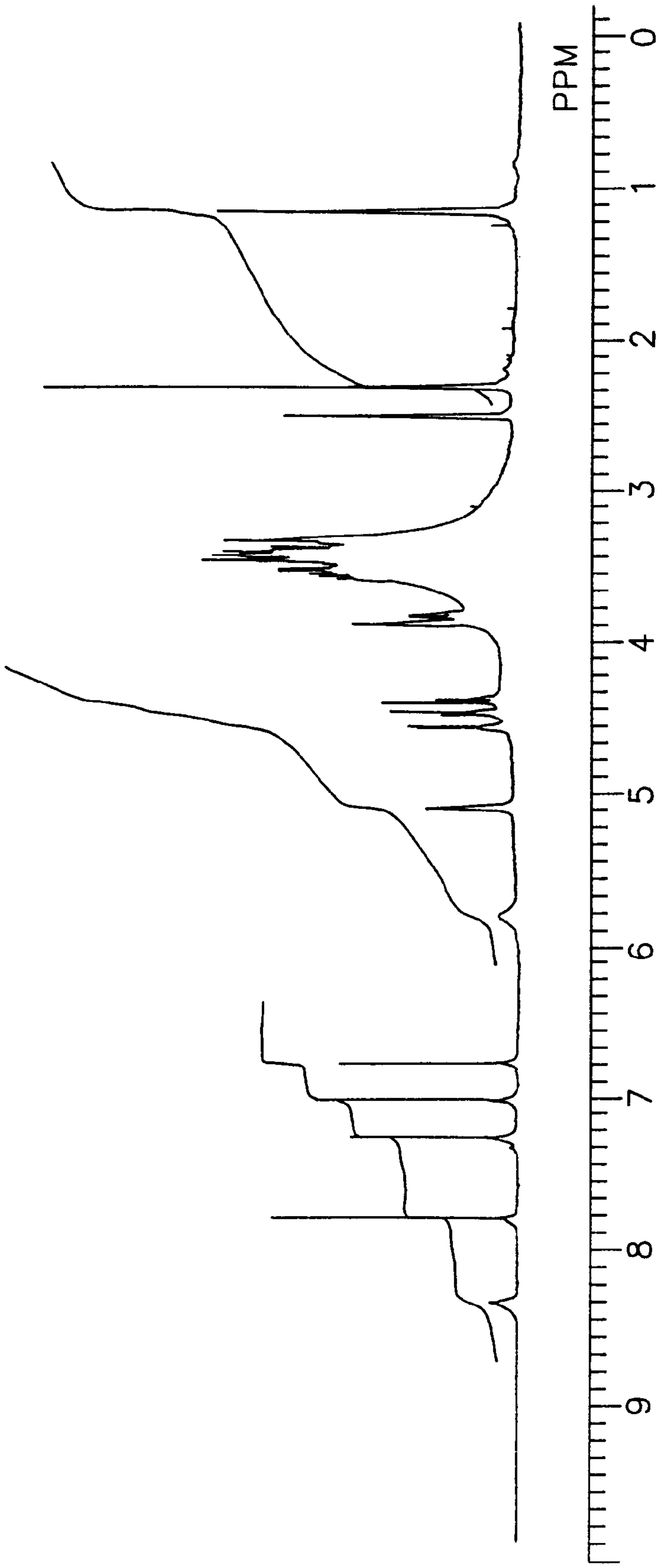


FIG.8

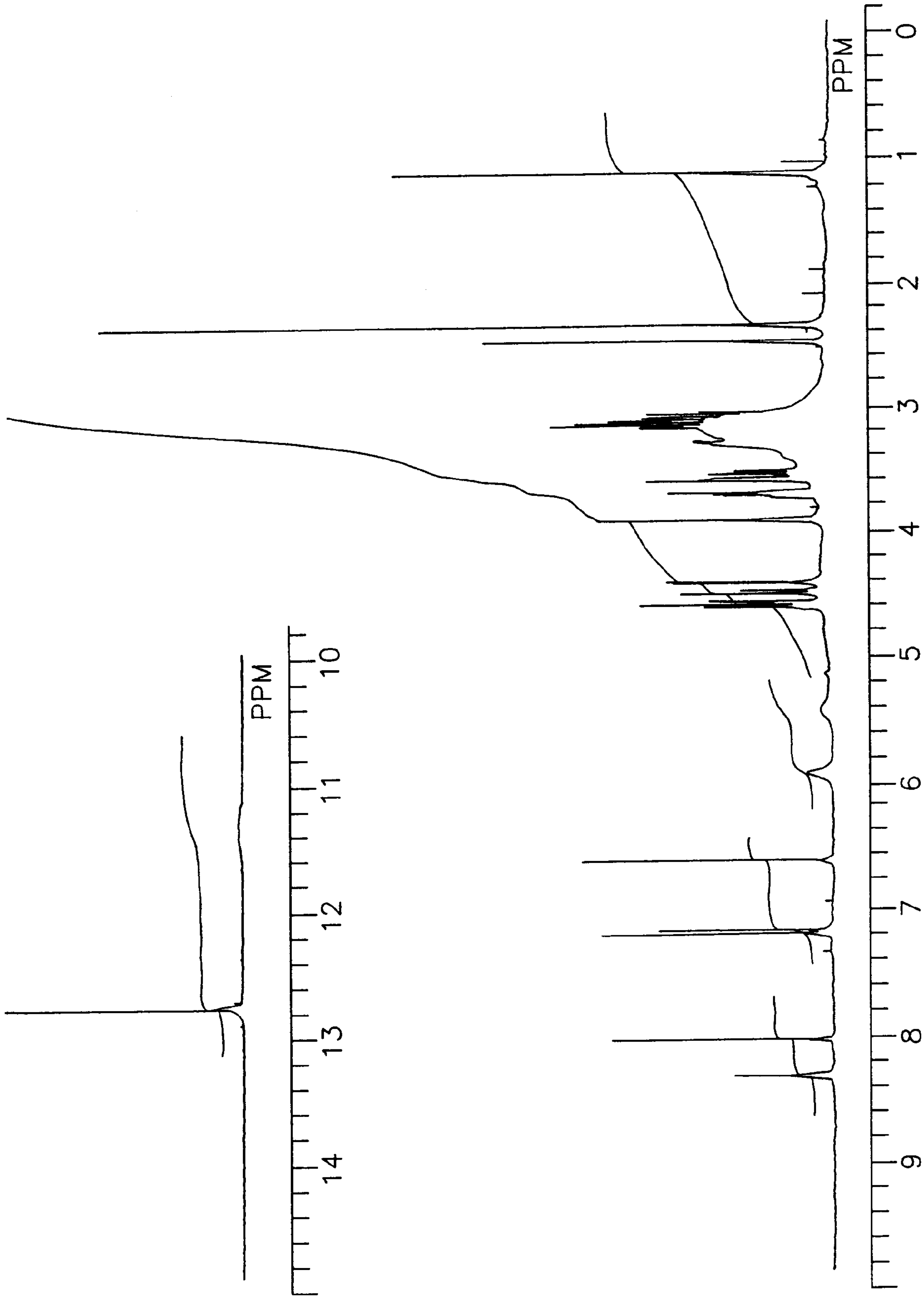


FIG.9

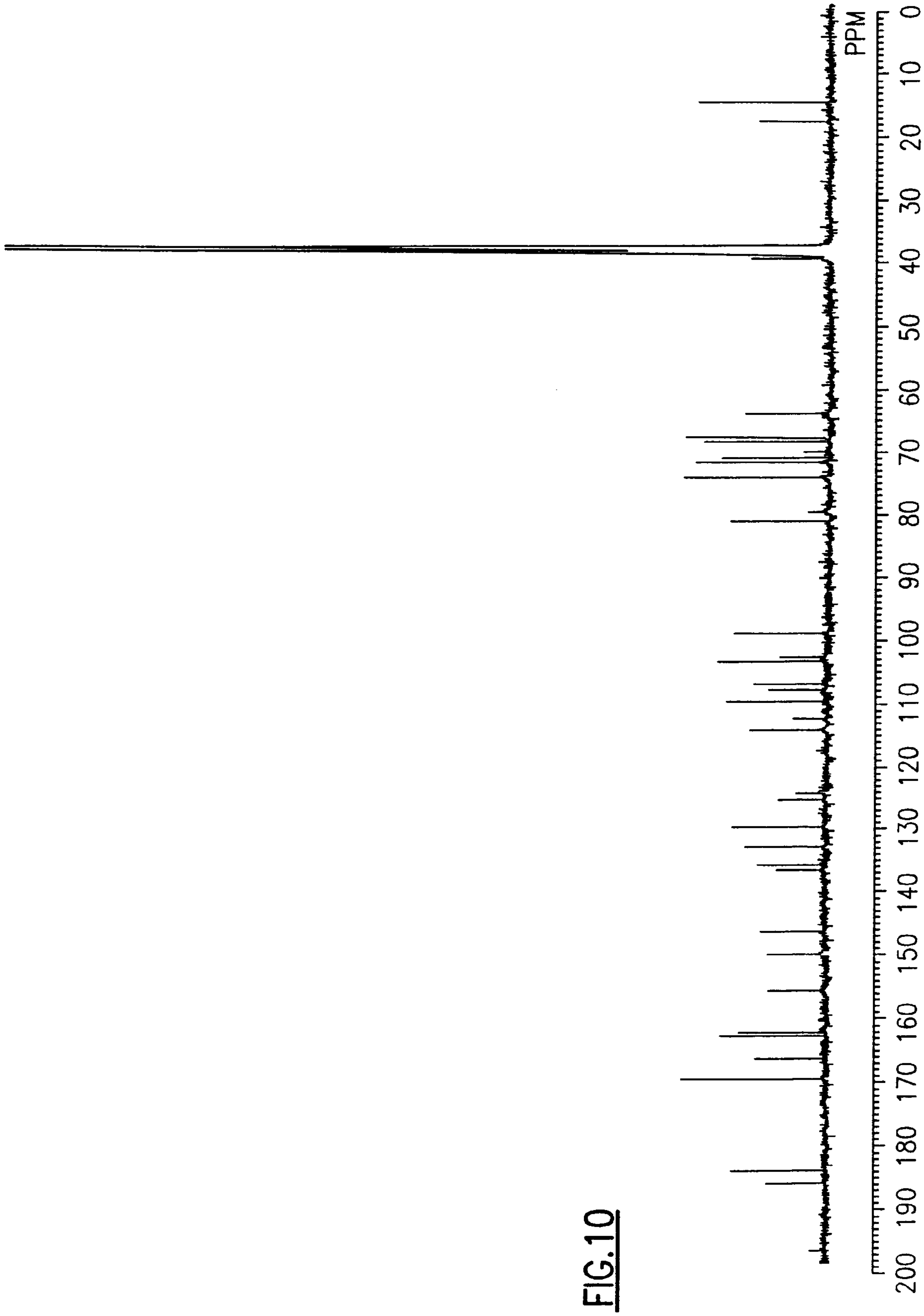
PPM
0
10
20
30
40
50
60
70
80
90
100
110
120
130
140
150
160
170
180
190
200

FIG.10

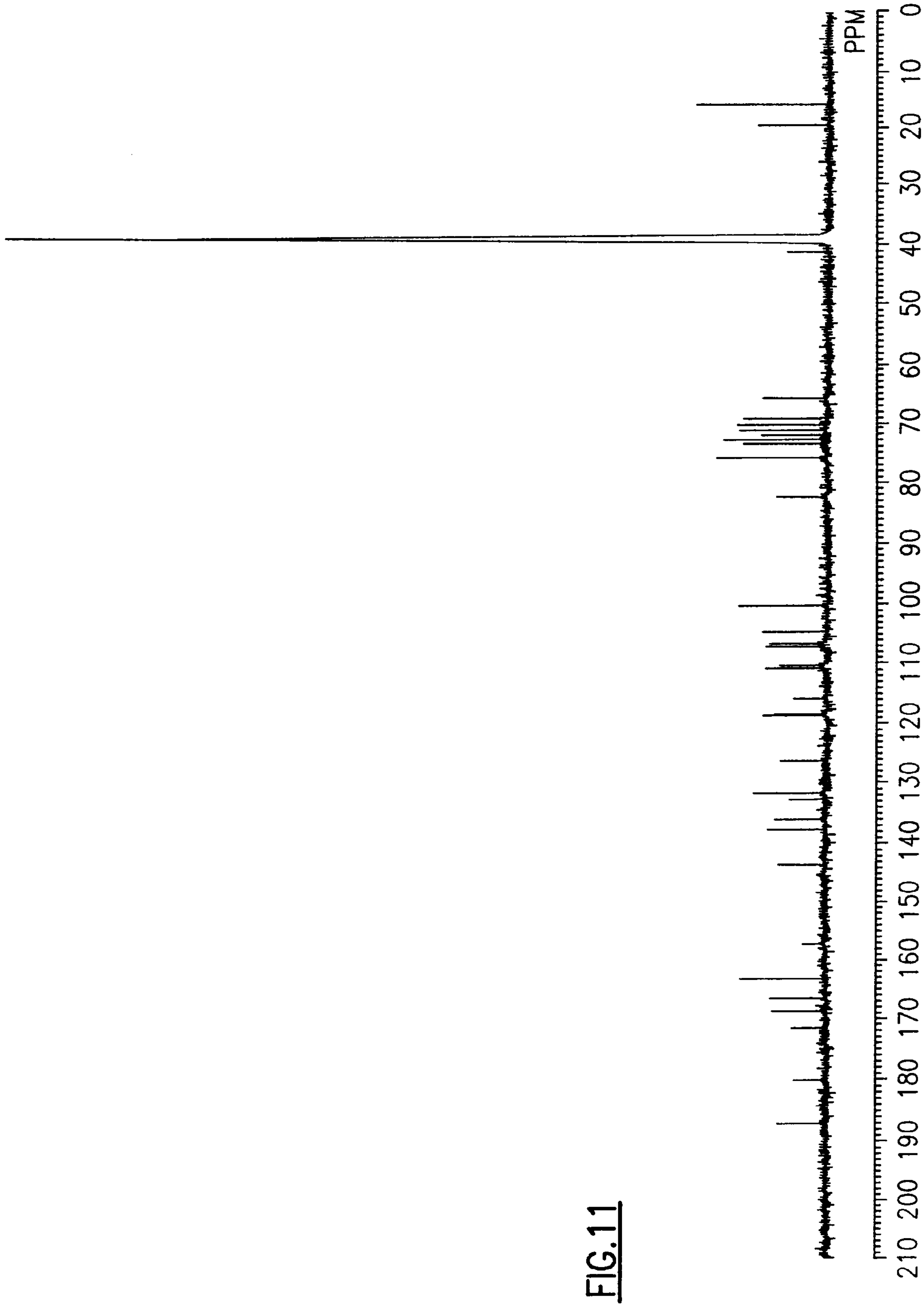
PPM
0
10
20
30
40
50
60
70
80
90
100
110
120
130
140
150
160
170
180
190
200
210

FIG.11

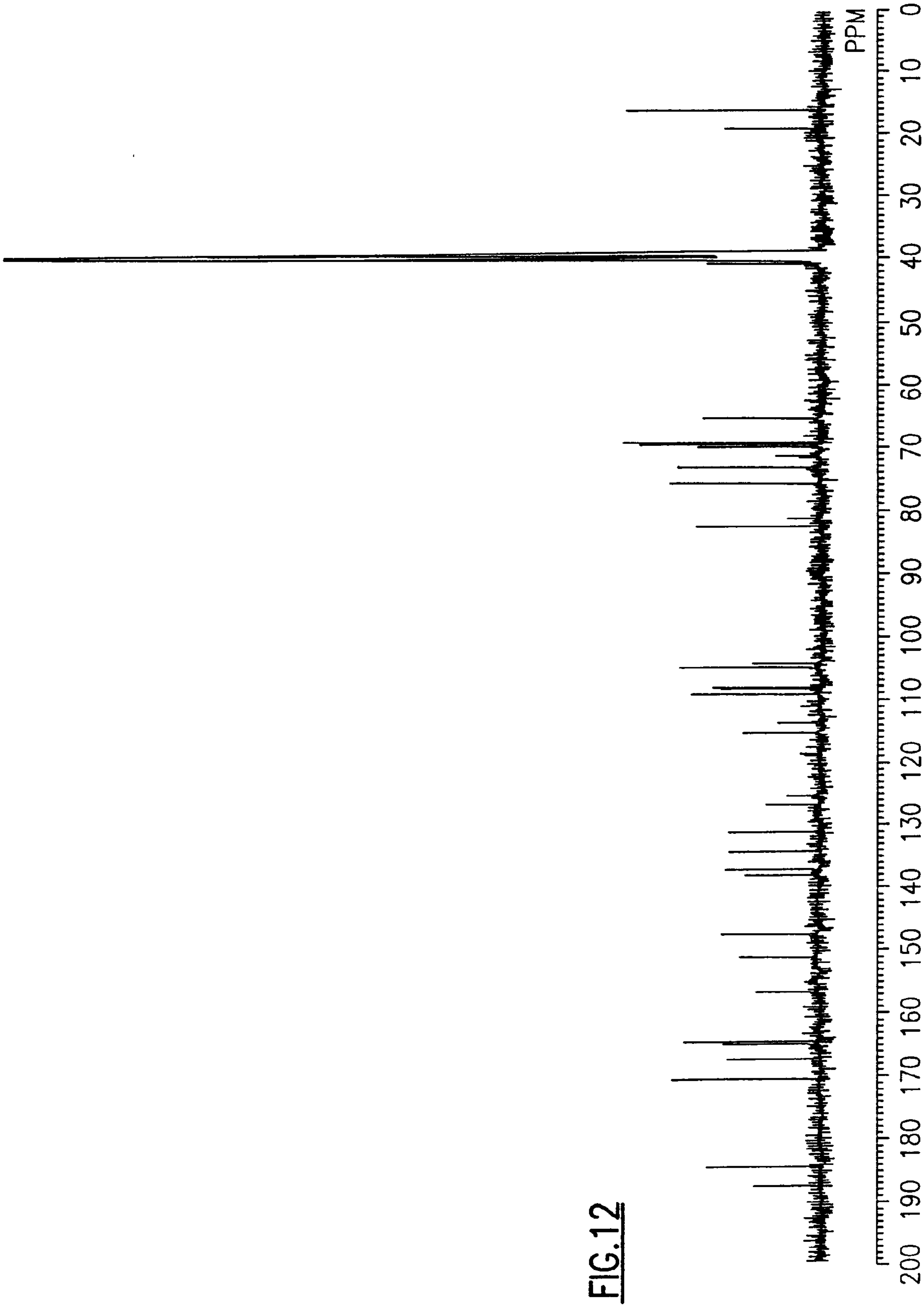
PPM
0
10
20
30
40
50
60
70
80
90
100
110
120
130
140
150
160
170
180
190
200

FIG.12

European Patent Office

# EUROPEAN SEARCH REPORT

Application Number
EP 93 40 1909

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.5) |
| A | EP-A-0 432 527 (BRISTOL-MYERS SQUIBB COMPANY)<br>* the whole document *<br>--- | 1 | C07H15/244<br>A61K31/71<br>C12P19/56<br>C12N1/20<br>//(C12N1/20, C12R1:04) |
| A | THE JOURNAL OF ANTIBIOTICS<br>vol. 44, no. 2 , 1 February 1991 , TOKYO, JP<br>pages 123 - 129<br>KONDO, S. ET AL 'Isolation of new minor benanomicins'<br>* the whole document *<br>--- | 1 | |
| A | EP-A-0 257 629 (HOECHST AKTIENGESELLSCHAFT)<br>* the whole document *<br>----- | 1 | |
| | | | TECHNICAL FIELDS SEARCHED (Int.Cl.5) |
| | | | C07H<br>A61K<br>C12P<br>C12R |

The present search report has been drawn up for all claims

| Place of search | Date of completion of the search | Examiner |
|---|---|---|
| THE HAGUE | 26 October 1993 | MORENO, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)